# EUROPEAN PATENT APPLICATION

(11) **EP 1 674 093 A1**
(43) Date of publication of application: **28.06.2006**
(21) Application number: 03754127.3
(22) Date of filing: 15.10.2003
(51) Int. Cl.: A61K 31/203, A61K 9/51, A61K 47/02, A61K 47/10, A61K 47/30, A61P 3/02

(54) **METHOD OF CONTROLLING PARTICLE SIZE OF RETINOIC ACID NANOPARTICLES COATED WITH POLYVALENT METAL INORGANIC SALT AND NANOPARTICLES OBTAINED BY THE CONTROLLING METHOD**

(71) Applicant: LTT Bio-Pharma Co., Ltd., Tokyo 105-6201 (JP)
(72) Inventor: YAMAGUCHI, Yoko 27-17, Ushijima, Kanagawa 258-0022 (JP); IGARASHI, Rie 8-2, Minamiikuta 5-chome, Kanagawa 214-0036 (JP); MIZUSHIMA, Yutaka 12-3-2402, Roppongi 6-chome, Tokyo 106-0032 (JP); TAKENAGA, Mitsuko 30-1, Sugao 2-chome, Kanagawa 216-0015 (JP); NAKAMURA, Natsumi 1-5-111, Tsuchihashi 7-chome, Kanagawa 216-0005 (JP)
(74) Representative: Albrecht, Thomas
(86) International application number: PCT/JP2003/013180
(87) International publication number: WO 2005/037267

(57) **Abstract**

Nanoparticles containing retinoic acid have reduced irritancy of retinoic acid and are suitable for subcutaneous or intravenous administration, as well as for use in sustained-release preparation.

The high skin permeability of the nanoparticles makes them suitable for use in pharmaceutical or non-pharmaceutical external preparations or cosmetics intended for skin application. The present invention provides a method for adjusting the particle size of such nanoparticles and nanoparticles produced by such a method. Specifically, the method involves dispersing retinoic acid dissolved in a lower alcohol in an aqueous alkali solution; adding a nonionic surfactant to the dispersion to form a mixed micelle; adding to the micelle a halide or acetate of divalent metal along with a carbonate or phosphate of alkali metal so that the molar ratio of the former to the latter is 1:0 to 1:1.0, thereby depositing a coating of inorganic salt of polyvalent metal on the surface of the micelle; and adjusting the average particle size of the resulting nanoparticles to 5 to 300 nm. The inorganic salt of polyvalent metal may be calcium carbonate, zinc carbonate, or calcium phosphate.

## Description

### TECHNICAL FIELD

The present invention relates to a method for adjusting the particle size of retinoic acid nanoparticles coated with an inorganic salt of polyvalent metal. More particularly, the present invention relates to a method for adjusting the particle size of retinoic acid nanoparticles coated with an inorganic salt of polyvalent metal, such as calcium carbonate, zinc carbonate, or calcium phosphate, as well as to nanoparticles obtained by the method.

### BACKGROUND ART

Retinoic acid, a liposoluble vitamin A acid, has recently attracted much attention for its ability to induce differentiation of embryonic stem (ES) cells and various other undifferentiated cells. Retinoic acid has been clinically used as a cure for acute promyuelocytic leukemia.

However, retinoic acid has irritancy due to carboxyl groups present in the molecule and, when subcutaneously administered, causes inflammation or tumor formation at the site of injection. Moreover, the high solubility of retinoic acid in lipids makes it difficult to formulate the compound into injections. Accordingly, various drug delivery systems (DDSs) have been proposed that are designed for sustained-release or targeted delivery of retinoic acid (See, for example, C. S. Cho, K. Y. Cho, I. K. Park, S. H. Kim, T. Sugawara, M. Uchiyama & T. Araike: *"Receptor-mediated delivery of all trans-retinoic acid to hepatocyte using poly(L-lactic acid) nanoparticles coated with galactose-carrying polystylene", J.*

### Control Release, 2001 Nov. 9:77(1-2), 7-15).

An injection has also been proposed that uses biodegradable polymer (See, for example, G. G. Giordano, M. FD. Refojo & M. H. Arroyo: *"Sustained delivery of retinoic acid from microsphers of biodegradable polymer in PVR", Invest. Ophthalmol. Vis., 1993 Aug. 34(9): 274-2745).*

Retinoic acid also has an ability to promote the growth of epithelial cells and, thus, the possibility of its use in cosmetics has been examined: The compound is expected to act to eliminate skin wrinkles and act as a skin-vitalizing or anti-aging agent (Japanese Patent Laid-open Publication No. Hei 09-503499). Nonetheless, the strong irritancy of retinoic acid, a common property of carboxylic acids, causes inflammation and other skin problems, making the compound unsuitable for use in cosmetics.

In an attempt to address these problems, the present inventors have previously proposed retinoic acid-containing nanoparticles that can be delivered subcutaneously or intravenously for sustained-release of the active ingredient. When applied to skin, the nanoparticles can elicit the advantageous effects of retinoic acid (See, for example, Japanese Patent Laid-Open Publication No. 2003-172493; *Journal of Pharmaceutical Science and Technology, Vol. 62 Mar. 2002, Supplement,* The Academy of Pharmaceutical Science and Technology, Japan, Abstracts of lectures of 17th annual meeting; *Drug Delivery System (DDS), Vol. 18, No. 3 May.:221 (2003); 29th Annual Meeting of the Controlled Release Society in Collaboration with the Korean Society for Biomaterials; Final Program July 20-25 (2002)).*

The previously proposed retinoic acid-containing nanoparticles are prepared in the following manner: Retinoic acid dissolved in a small amount of a polar solvent is dispersed in alkali-containing water. To this dispersion, a nonionic surfactant is added to form mixed micelles, to which a salt of divalent metal is added, followed by a salt that can form negative divalent ion. This gives the desired product.

The retinoic acid-containing nanoparticles so prepared comprise particles that have a coating of a metal compound deposited on the surface thereof. For example, when the salt of divalent metal is calcium chloride and the salt that can form negative divalent ion is sodium carbonate, a coating of calcium carbonate is deposited on the surface of nanoparticles.

The retinoic acid-containing nanoparticles previously provided by the present inventors are prepared by taking advantage of the amphipathic property of retinoic acid. Specifically, retinoic acid is first dispersed in an aqueous solution to form spherical micelles having negatively charged surface. A nonionic surfactant and then calcium chloride are added to allow calcium ion (Ca²⁺) to adsorb onto the negatively charged micelle surface. This prevents aggregation and subsequent precipitation of retinoic acid micelles and gives spherical or oval micelles covered with calcium ions. Sodium carbonate is then added to allow carbonate ion (CO₃²⁻) to adsorb onto (bind to) the calcium ion-coated micelle surface and thus completely neutralize the surface charge of the micelles. As a result, calcium carbonate coating is deposited on the surface of the retinoic acid micelles, thus giving the desired calcium carbonate-coated retinoic acid nanoparticles.

As opposed to calcium carbonate crystals obtained by the precipitation method or the uniform precipitation method, which are substantially water-insoluble crystals commonly known as calcite, the calcium carbonate deposited on the spherical or oval micelle surface by the above-described process is not likely to form hard crystals, but rather has a glass-like amorphous structure or metastable vaterite structure. If the calcium carbonate coating has amorphous structure, which unlike hard crystal structure, has a high solubility in water and is highly biodegradable, the coating is readily decomposed. Similarly, the coating formed as a vaterite is readily biodegraded since vaterite has a higher solubility in water than the other crystalline forms of calcium carbonate: calcite and aragonite.

Thus, the calcium carbonate-coated retinoic acid nanoparticles obtained by the above-describe process, when administered to a living body, have a sustained effect as the calcium carbonate layer on the micelle surface is degraded to release retinoic acid contained in the micelles.

Aside from calcium carbonate, other biocompatible inorganic salts of polyvalent metals, such as zinc carbonate and calcium phosphate, may be used to coat the surface of the micelles containing retinoic acid to achieve the same effect.

One problem is that the retinoic acid nanoparticles coated with calcium carbonate or other inorganic salts of polyvalent metal have a varying particle size (diameter) of 5 to 1000 nm and it has been considered difficult to effectively prepare nanoparticles of desired size: It is preferred that the nanoparticles are very small, specifically approximately 5 to 300 nm in size, for subcutaneous, intravenous, or topical application (transdermal application) of retinoic acid.

Accordingly, it is an object of the present invention to provide a method for adjusting the particle size, wherein the method enables preparation of very small retinoic acid nanoparticles coated with an inorganic salt of polyvalent metal, such as calcium carbonate, and sized approximately 5 to 300 nm.

In an effort to achieve this object, the present inventors have found that by adjusting the molar ratio of the metal halide or metal acetate to the alkali metal carbonate or alkali metal phosphate added during deposition of the coating of inorganic salt of polyvalent metal on the surface of retinoic acid micelles, the coated retinoic acid particles having an average particle of approximately 5 to 300 nm can be obtained. It is this discovery that led to the present invention.

### DISCLOSURE OF THE INVENTION

Accordingly, basic embodiments of the present invention comprise the following:
(1) A method for adjusting a particle size of retinoic acid nanoparticles comprising micelles of retinoic acid coated with an inorganic salt of polyvalent metal, the method comprising:
   dispersing retinoic acid dissolved in a lower alcohol in an aqueous alkali solution;
   adding a nonionic surfactant to the dispersion to form a mixed micelle;
   adding to the micelle a halide or acetate of divalent metal along with a carbonate or phosphate of alkali metal so that a molar ratio of the former to the latter is 1:0 to 1:1.0, thereby depositing a coating of the inorganic salt of the polyvalent metal on a surface of the micelle; and
   adjusting an average particle size of the resulting nanoparticles to 5 to 300 nm.
(2) The method for adjusting a particle size of retinoic acid nanoparticles coated with an inorganic salt of polyvalent metal according to (1) above, wherein the coating of the inorganic salt of the polyvalent metal is calcium carbonate, zinc carbonate, or calcium phosphate.
(3) The method for adjusting the particle size of retinoic acid nanoparticles coated with an inorganic salt of polyvalent metal according to (1) above, wherein the halide or acetate of divalent metal is calcium halide, zinc halide, calcium acetate or zinc acetate.
(4) The method for adjusting the particle size of retinoic acid nanoparticles coated with an inorganic salt of polyvalent metal according to (3) above, wherein the calcium halide or the zinc halide is selected from the group consisting of calcium chloride, calcium bromide, calcium fluoride, calcium iodide, zinc chloride, zinc bromide, zinc fluoride and zinc iodide.
(5) The method for adjusting the particle size of retinoic acid nanoparticles coated with an inorganic salt of polyvalent metal according to (1) above, wherein the carbonate or phosphate of alkali metal is selected from the group consisting of sodium carbonate, potassium carbonate, sodium phosphate, and potassium phosphate.
(6) The method for adjusting the particle size of retinoic acid nanoparticles coated with an inorganic salt of polyvalent metal according to (1) above, wherein the lower alcohol is methanol or ethanol.
(7) The method for adjusting the particle size of retinoic acid nanoparticles coated with an inorganic salt of polyvalent metal according to (1) above, wherein the nonionic surfactant is polyoxyethylene (20) sorbitan monooleate, polyoxyethylene (20) sorbitan monolaurate, polyoxyethylene (20) sorbitan monostearate, polyoxyethylene (20) sorbitan monopalmitate, polyoxyethylene (20) sorbitan trioleate, polyoxyethylene (8) octylphenylether, polyoxyethylene (20) cholesterol ester, polyoxyethylene (30) cholesterol ester or polyoxyethylene hydrogenated castor oil.
   More specific embodiments of the present invention comprise the following:
(8) The method for adjusting a particle size of retinoic acid nanoparticles coated with calcium carbonate claimed in claim 1, comprising micelles of retinoic acid coated with calcium carbonate, the method comprising:
   dispersing retinoic acid dissolved in a lower alcohol in an aqueous alkali solution;
   adding a nonionic surfactant to the dispersion to form a mixed micelle;
   adding to the micelle calcium chloride and sodium carbonate so that a molar ratio of the former to the latter is 1:0 to 1:1.0, thereby depositing a coating of calcium carbonate on a surface of the micelle; and
   adjusting the average particle size of the resulting nanoparticles to 5 to 300 nm.
(9) The method for adjusting the particle size of retinoic acid nanoparticles according to (8) above, wherein the lower alcohol is methanol or ethanol.
(10) The method for adjusting the particle size of retinoic acid nanoparticles coated with calcium carbonate according to (8) above, wherein the nonionic surfactant is polyoxyethylene (20) sorbitan monooleate, polyoxyethylene (20) sorbitan monolaurate, polyoxyethylene (20) sorbitan monostearate, polyoxyethylene (20) sorbitan monopalmitate, polyoxyethylene (20) sorbitan trioleate, polyoxyethylene (8) octylphenylether, polyoxyethylene (20) cholesterol ester, polyoxyethylene (30) cholesterol ester or polyoxyethylene hydrogenated castor oil.
   Other embodiments of the present invention comprise the following:
(11) Retinoic acid nanoparticles coated with inorganic salt of polyvalent metal and having an average particle size of 5 to 300 nm, obtained by the adjusting method according to any of claims (1) through (7).
(12) Calcium carbonate-coated retinoic acid nanoparticles obtained by the adjusting method according to any of claims (8) through (10) and having an average particle size of 5 to 300 nm.
(13) Calcium carbonate-coated nanoparticles having an average particles size of 5 to 300 nm and comprising retinoic acid micelles coated with calcium carbonate.
(14) Zinc carbonate-coated retinoic acid nanoparticles having an average particles size of 5 to 300 nm and comprising retinoic acid micelles coated with zinc carbonate.
(15) Calcium phosphate-coated retinoic acid nanoparticles having an average particles size of 5 to 300 nm and comprising retinoic acid micelles coated with calcium phosphate.

The retinoic acid nanoparticles coated with an inorganic salt of polyvalent metal provided in accordance with the present invention have a very small average particle size adjusted to a desired range of 5 to 300 nm.

Retinoic acid is a highly irritant and lipophilic compound and can thus cause inflammation and tumor formation at the site of application when subcutaneously administered. Moreover, the insolubility of retinoic acid in water makes it unsuitable for use in injections. On the other hand, the retinoic acid nanoparticles coated with an inorganic acid of polyvalent metal of the present invention can be dissolved in water to form a clear solution, which remains clear when left and can thus be formulated into injection preparations for subcutaneous and intravenous administration. The inorganic salt coating is biocompatible and helps reduce the irritancy of retinoic acid, so that the nanoparticles do not cause inflammation or tumor formation at the site of application.

In addition, when applied to skin as an external preparation, the nanoparticles of the present invention are percutaneously absorbed, yet cause no inflammation because of less irritancy. The nanoparticles then release retinoic acid in a sustained manner, acting to eliminate skin wrinkles and activate skin. Thus, the nanoparticles of the present invention find applications in external preparations and cosmetics.

Retinoic acid is particularly effective when applied to skin: It promotes the growth of epithelial cells and facilitates the regeneration of the skin. Although these advantageous properties have led to the expectation that the compound can be used for the purposes of skin beauty and wrinkle elimination, its skin irritancy has prevented the use of retinoic acid in these applications. With the coating of inorganic salt of polyvalent metal, however, the nanoparticles of the present invention have significantly reduced irritancy. Moreover, the small average particle size of 5 to 300 nm helps improve the skin permeability of the particles and facilitates the diffusion of retinoic acid into blood, so that the blood level of retinoic acid quickly reaches the effective point and remains there for an extended period of time.

This increases the production of HB-epidermal growth factor (HB-EGF) and induces the production of hyaluronic acid, which otherwise is not produced in epidermis in a short time. As a result, the regeneration of the skin is significantly accelerated, as is the thickening of epidermis. Thus, the nanoparticles of the present invention are highly useful in regenerative medicine.

### BRIEF DESCRIPTION OF THE DRAWINGS

Fig. 1 is a diagram showing the change in the transmittance of the solution by addition of sodium carbonate according to (1) of Test Example 1.
Fig. 2 is a diagram showing the change in zeta potential of retinoic acid-CaCO₃ particles prepared in (2) of Test Example 1 by mixing retinoic acid micelles with sodium carbonate and calcium chloride while the molar ratio of sodium carbonate to calcium chloride was varied.
Fig. 3 is a diagram showing ³H-thymidine uptake by melanoma cells stimulated by retinoic acid in accordance with Test Example 5.
Fig. 4 is a diagram showing the change in the blood level of retinoic acid when retinoic acid-CaCO₃ nanoparticles and retinoic acid micelles were subcutaneously administered to rats according to (1) of Test Example 6.
Fig. 5 is a photograph showing the site of application 10 days after the retinoic acid micelles (not formulated as nanoparticles) were subcutaneously administered to rats according to (1) of Comparative Example 6.
Fig. 6 is a photograph showing the site of application 10 days after the retinoic acid-CaCO₃ nanoparticles were subcutaneously administered to rats according to (1) of Test Example 6.
Fig. 7 is a diagram showing the change in the blood level of retinoic acid when retinoic acid-CaCO₃ nanoparticles, retinoic acid-ZnCO₃ nanoparticles and retinoic acid were mixed with a Vaseline base and were individually applied to the skin of mice according to (2) of Test Example 6.
Fig. 8 is a diagram showing the thickness of epidermis in mice administered different preparations according to Test Example 7.
Fig. 9 is a diagram showing a comparison of expression levels of HB-EGF mRNA according to Test Example 8.

Throughout the diagrams, RA indicates retinoic acid; RA-CaCO₃ indicates retinoic acid-CaCO₃ nanoparticles; RA-ZnCO₃ indicates retinoic acid-ZnCO₃ nanoparticles; RA-Ca indicates retinoic acid-Ca nanoparticles; and RA-Zn indicates retinoic acid-Zn nanoparticles.

### BEST MODE FOR CARRYING OUT THE INVENTION

Retinoic acid for use in the present invention is all-trans retinoic acid, a compound involved in various physiological functions, including proper functioning of vision, auditory sense and reproductive functions, maintenance of skin and mucosa and suppression of cancer. All-trans retinoic acid has been clinically used in the treatment of acute promyelocytic leukemia (APL).

Specifically, the retinoic acid nanoparticles coated with an inorganic salt of a polyvalent metal are prepared as described below.

A lipophilic compound with carboxyl groups in its molecule, retinoic acid forms spherical micelles in an aqueous alkali solution, such as aqueous sodium hydroxide solution containing a small amount of a lower alcohol. The surface of the micelle is negatively charged and readily adsorbs (binds to) divalent metal ion, such as calcium ion (Ca²⁺), replacing sodium ion. Since the divalent metal ion is more tightly adsorbed (bound) to the micelles than is the sodium ion, the micelles having the divalent metal ions adsorbed on them have more stable surface charge, so that they become insoluble in water and precipitate. The precipitated particles aggregate into large clusters.

To prevent aggregation of the charged particles, a nonionic surfactant, such as polyoxyethylene (20) sorbitan monooleate (Tween 80), is added along with retinoic acid. Tween 80, together with retinoic acid, forms mixed micelles that have polyoxyethylene chains sticking out from their surface. The presence of the hydrophilic polyoxyethylene chains on the micelle surface prevents the precipitation of the micelles when they adsorb (bind to) polyvalent metal ions.

A halide or acetate of a divalent metal, such as calcium chloride, is then added in a sufficiently large amount so that the divalent metal ions can adsorb onto the surface of the retinoic acid micelles. The divalent metal ions are more tightly adsorbed (bound) to the micelle surface than sodium ions and thus replace sodium ions on the micelle surface. The primarily adsorbed (bound) divalent metal ions cover the micelle surface to form spherical or oval micelles. A carbonate or phosphate of an alkali metal is then added to the system to allow carbonate ions (CO₃²⁻) or phosphate ions (PO₄²⁻) to adsorb onto the divalent metal ions on the still unneutralized micelle surface. These results in the deposition of a coating of an inorganic salt of polyvalent metal on the surface of the retinoic acid micelles, thus giving the desired retinoic acid nanoparticles coated with an inorganic salt of polyvalent metal.

The inorganic salt of a polyvalent metal that coats the nanoparticles of the present invention may be calcium carbonate, zinc carbonate or calcium phosphate, each a biocompatible salt.

Thus, the halide or acetate of divalent metal is calcium halide, zinc halide, calcium acetate or zinc acetate. Specific examples of the calcium halide and zinc halide include calcium chloride, calcium bromide, calcium fluoride, calcium iodide, zinc chloride, zinc bromide, zinc fluoride and zinc iodide.

Examples of the alkali metal carbonate or alkali metal phosphate include sodium carbonate, potassium carbonate, sodium phosphate and potassium phosphate.

The lower alcohol for use in the preparation of the nanoparticles may be methanol or ethanol.

Examples of the nonionic surfactant include polyoxyethylene (20) sorbitan monooleate (Tween 80), polyoxyethylene (20) sorbitan monolaurate (Tween 20), polyoxyethylene (20) sorbitan monostearate (Tween 60), polyoxyethylene (20) sorbitan monopalmitate (Tween 40), polyoxyethylene (20) sorbitan trioleate (Tween 85), polyoxyethylene (8) octylphenylether, polyoxyethylene (20) cholesterol ester, and polyoxyethylene hydrogenated castor oil.

While the coated retinoic acid nanoparticles prepared by the above-described method are very small, they have a wide particle size distribution ranging from about 10 to about 3000 nm (in diameter).

It is preferred that the coated retinoic acid nanoparticles have a very small size of about 5 to about 300 nm when they are intended for subcutaneous, intravenous or topical application (transdermal application). Thus, the size of the desired retinoic acid nanoparticles coated with an inorganic acid of polyvalent metal must be adjusted to about 5 to about 300 nm.

It has turned out that the size of the nanoparticles can be adjusted to the desired range by varying the amounts of the components for depositing the coating on the surface of the retinoic acid micelle, specifically by varying the molar ratio of the halide or acetate of divalent metal to the carbonate or phosphate of alkali metal.

Specifically, the coating of the polyvalent metal inorganic salt is deposited on the surface of the retinoic acid micelles by exchanging the negative charge imparted to the micelle surface in the alkali (i.e., sodium) solution for the divalent metal ion resulting from the halide or acetate of divalent metal and by neutralizing the negative charge with the carbonate ion (CO3²⁻) or phosphate ion (PO4²⁻) resulting from the carbonate or phosphate of alkali metal.

In particular, by adjusting the molar ratio of the halide or acetate of divalent metal to the carbonate or phosphate of alkali metal within the range of 1:0 to 1:1.0, the coating of polyvalent metal inorganic salt is deposited on the micelle surface and the average particle size of the resulting nanoparticles is adjusted within the range of 5 to 300 nm. If necessary, the particles may be mechanically shaken by for example ultrasonication.

If 1.0mol or more of the carbonate or phosphate of alkali metal is added per 1mol of the halide or acetate of divalent metal, the resulting particles will become excessively large in size though the micelle surface may be properly coated with the inorganic salt of polyvalent metal. As a result, the particles aggregate with each other, so that the nanoparticles with the desired average particle size can no longer be obtained even by mechanically shaking them by, for example, ultrasonication.

Conversely, if the molar ratio of the halide or acetate of divalent metal to the carbonate or phosphate of alkali metal is within the range of 1:0 to 1:1.0, then not only are the micelles properly coated with the inorganic salt of polyvalent metal, but the resulting nanoparticles have an average particle size of 5 to 300 nm.

The resulting nanoparticles may form aggregates. It has proven that such aggregates can be mechanically shaken by, for example, ultrasonication to obtain nanoparticles highly uniform in size. Thus, such aggregates are also encompassed by the scope of the present invention and a method of the present invention may involve mechanical shaking of the resulting nanoparticles, such as ultrasonication, to adjust the average particle size of the nanoparticles.

The so prepared retinoic acid nanoparticles coated with an inorganic salt of polyvalent metal provided in accordance with the present invention can be dissolved in water to form a stable clear solution and causes less irritancy since retinoic acid is coated with the polyvalent metal inorganic salt. The nanoparticles can thus be formulated into injection preparations for subcutaneous or intravenous administration. In addition, the nanoparticles do not cause inflammation or tumor formation at the site of application.

Moreover, when applied to skin as an external preparation, the nanoparticles of the present invention are percutaneously absorbed, yet cause no inflammation because of less irritancy. The nanoparticles then release retinoic acid in a sustained manner, acting to eliminate skin wrinkles and activate skin.

The present invention, including the above-described aspects, will now be described in detail with reference to test examples.

In the following description of the retinoic nanoparticles coated with an inorganic salt of polyvalent metal provided in accordance with the present invention, the retinoic acid nanoparticles coated with calcium carbonate may be referred to as "retinoic acid-CaCO₃ nanoparticles," the retinoic acid nanoparticles coated with zinc carbonate may be referred to as "retinoic acid-ZnCO₃ nanoparticles," and the retinoic acid nanoparticles coated with calcium phosphate may be referred to as "retinoic acid-CaPO₄ nanoparticles."

### Test Example 1: Preparation of retinoic acid-CaCO₃ nanoparticles

13.6mg retinoic acid was dissolved in 900µl ethanol (or methanol). To this solution, 100µl of 0.5N aqueous NaOH solution were added. The pH of the mixture was 7 to 7.5. A 100µl portion of the mixture was then added to 100µl distilled water containing Tween 80 and the resulting mixture was thoroughly stirred.

After approximately 30 min, a 5M aqueous calcium chloride solution was added and the mixture was stirred for another 30 min. Subsequently, a 1M aqueous sodium carbonate solution was added and the mixture was further stirred. The mixture was continuously stirred over one day and night, and the resulting solution was freeze-dried over night to give desired calcium carbonate-coated retinoic acid nanoparticles.

For testing, the freeze-dried retinoic acid-CaCO₃ nanoparticles were dispersed in injectable distilled water to a predetermined concentration.

### (1) Change in the transparency of the solution by addition of sodium carbonate

During the addition of the 5M aqueous calcium chloride solution and the 1M aqueous sodium carbonate solution described above, the amount of the 1M sodium carbonate solution was varied relative to a fixed amount (15µl) of the 5M calcium chloride solution to make different solutions containing sodium carbonate at varying molar ratios relative to calcium chloride. The change in the transmittance of the solution was observed.

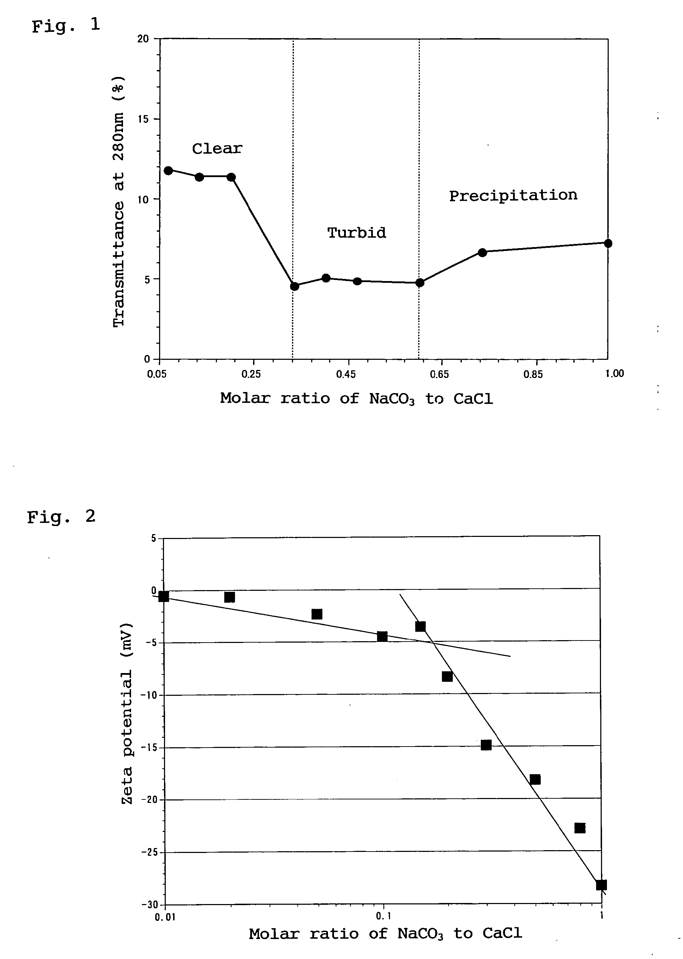

Specifically, the transmittance at 280 nm was determined for each solution (the higher the value of transmittance, the higher the transparency of the solution). The results are shown in Fig. 1.

As can be seen from the results shown in the figure, the solutions gradually became turbid as the amount of sodium carbonate was increased. Ultimately, precipitation was observed. Each of the solutions that formed precipitation remained clear or slightly turbid until 30min after the addition of sodium carbonate, but further stirring caused the precipitation to form, resulting in an increased turbidity of the supernatant.

These observations indicate that the nanoparticles of desired particle size can be obtained by varying the molar ratios of calcium chloride and sodium carbonate relative to the retinoic acid micelles.

### (2) Change in zeta potential by varying molar ratio of sodium carbonate to calcium chloride

Retinoic acid-CaCO₃ nanoparticles were prepared with varying molar ratios of sodium carbonate to calcium chloride, and the change in zeta potential was determined.

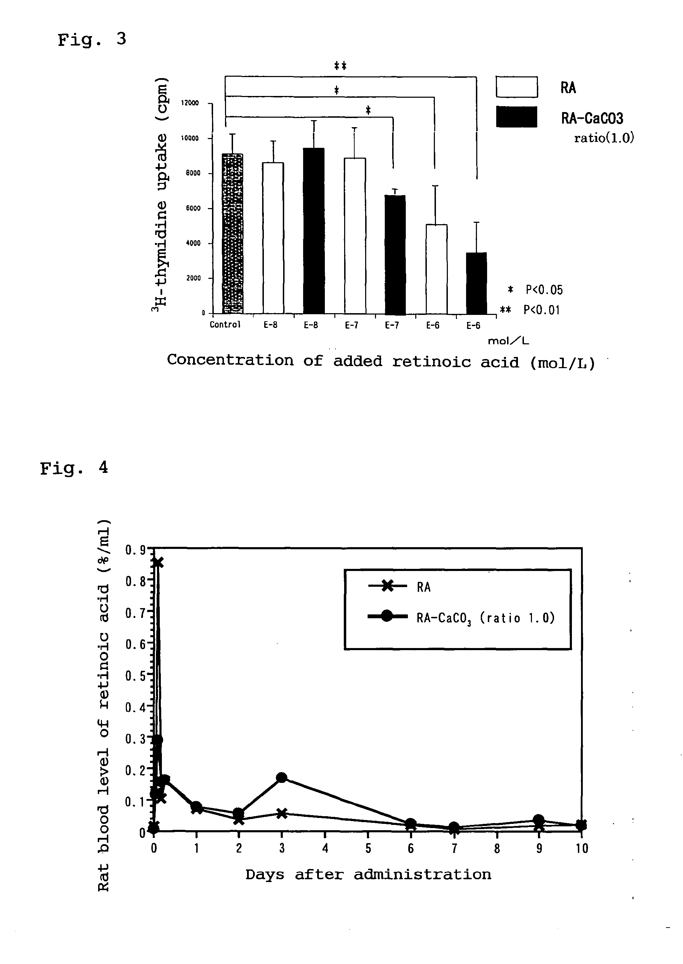

The results are shown in Fig. 2.

Retinoic acid micelles in the presence of calcium chloride only showed a zeta potential of +1.66 mV, while retinoic acid micelles alone showed a zeta potential of -68.5 mV, a clear indication that calcium ions were adsorbed onto the micelle surface.

As can be seen from the results of Fig. 2, the zeta potential of the nanoparticles varied as the molar ratio of sodium carbonate to calcium chloride was varied. As shown, zeta potential slowly decreased when the amount of sodium carbonate added per one mol of calcium chloride was 0.2 mols or less: It decreased rapidly when the amount of sodium carbonate exceeded 0.2 mol per one mol of calcium chloride. This indicates that either the adsorption of carbonate ions has reached saturation at this molar ratio or the particles have started aggregating with each other.

### Test Example 2: The effect of the molar ratio of sodium carbonate to calcium chloride on the particle size of retinoic acid-CaCO₃ nanoparticles

### (1) The molar ratio

The results of Test Example 1 demonstrate that the particle size of-retinoic acid-CaCO₃ nanoparticles can be adjusted by varying the molar ratios of calcium chloride and sodium carbonate added to the micelles of retinoic acid.

We next examined the change in the particle size of retinoic acid-CaCO₃ nanoparticles by varying the molar ratios of calcium chloride and sodium carbonate added to the micelles of retinoic acid.

It turned out that the resulting retinoic acid-CaCO₃ nanoparticles were 10 to 50 nm in size when the amount of sodium carbonate added per one mol of calcium chloride was 0.2 mols or less.

When the amount of sodium carbonate added per one mol of calcium chloride was greater than 0.2 mols, the resultant retinoic acid-CaCO₃ nanoparticles were 350 nm or larger in size. The extremely large average particle size indicates that the nanoparticles had aggregated into large clusters.

This observation is consistent with the change in zeta potential observed in (2) of Test Example 1.

We next determined if these aggregates could be dispersed into fine nanoparticles by sonication.

### (2) The fluid dynamically determined size of the ultrasonicated retinoic acid-CaCO₃ nanoparticles

The retinoic acid-CaCO₃ nanoparticles obtained in the manner described above were ultrasonicated for 5 min and the particle size was determined.

It was demonstrated that when the amount of sodium carbonate added per one mol of calcium chloride was 1.0mol or less, the resulting aggregates of retinoic acid-CaCO₃ nanoparticles could be dispersed by ultrasonication into nanoparticles smaller than 300 nm in size. In comparison, the aggregates of the retinoic acid-CaCO₃ nanoparticles obtained by adding 2.0 mols and 3.0 mols of sodium carbonate per one mol of calcium chloride (approximately 2700 nm and 2200 nm in average particle size, respectively) could not be dispersed by ultrasonication.

The results are together shown in Table 1.

**Table 1 The effect of the molar ratio of calcium chloride to sodium carbonate on the particle size of nanoparticles**

| | Average particle size of retinoic acid-CaCO₃ nanoparticles (nm) | | | | | | | |
|---|---|---|---|---|---|---|---|---|
| Molar ratio of CaCl₂/NaCO₃ | 1/0 | 1/0.01 | | 1/0.1 | | 1/0.2 | | 1/0.3 |
| Average particle size immediately after preparation (nm) | 20.2 | 23 | | 17.3 | | 30.3 | | 356.3 |
| Average particle size after ultrasonication (nm) | - | - | | - | | 22.4 | | 33.3 |
| Molar ratio of CaCl₂/NaCO₃ | 1/0.5 | | 1/1.0 | | 1/2.0 | | 11/3.0 | |
| Average particle size immediately after preparation (nm) | 1316.2 | | 1450 | | 2687.3 | | 2240.5 | |
| Average particle size after ultrasonication (nm) | 41.1 | | 106.4 | | 2197.3 | | 1349.4 | |

These observations suggest that the average particle size of the retinoic acid-CaCO₃ nanoparticles can be effectively adjusted by adding calcium chloride and sodium carbonate so that the molar ratio of the former to the latter is 1:0 to 1:1.0 and the calcium carbonate coating is thereby deposited on the surface of retinoic acid micelles, and then ultrasonicating the resulting nanoparticles.

A freeze-fracture transmittance electron microscopy (ff-TEM) of the size-adjusted retinoic acid-CaCO₃ nanoparticles, obtained by adding calcium chloride and sodium carbonate at a molar ratio of 1:0.2 to deposit calcium carbonate coating on the surface of retinoic acid micelles, has revealed that the retinoic acid-CaCO₃ nanoparticles have a core-shell structure, as seen in the fractured surface.

This implies that the structure consists of the retinoic acid core surrounded by the shell of calcium carbonate.

### Test Example 3: Preparation of retinoic acid-ZnCO₃ nanoparticles

13.6mg retinoic acid was dissolved in 900µl ethanol. To this solution, 100µl of 0.5N aqueous NaOH solution were added. The pH of the mixture was 7 to 7.5. A 100µl portion of the mixture was added to 100µl distilled water containing Tween 80 and the resulting mixture was thoroughly stirred.

After approximately 30 min, a 5M aqueous zinc acetate solution was added and the mixture was stirred for another 30 min. Subsequently, a 1M aqueous sodium carbonate solution was added and the mixture was further stirred. The mixture was continuously stirred over one day and night, and the resulting solution was freeze-dried over night to give desired zinc carbonate-coated retinoic acid nanoparticles (retinoic acid-ZnCO₃ nanoparticles).

The resulting retinoic acid-ZnCO₃ nanoparticles were similar to the nanoparticles of Test Examples 1 and 2 in terms of particle size.

For testing, the freeze-dried retinoic acid-ZnCO₃ nanoparticles were dispersed in injectable distilled water to a predetermined concentration.

### Test Example 4: Preparation of retinoic acid-CaPO₄ nanoparticles

13.6mg retinoic acid was dissolved in 900µl ethanol. To this solution, 100µl of 0.5N aqueous NaOH solution were added. The pH of the mixture was 7 to 7.5. A 100µl portion of the mixture was then added to 100µl distilled water containing Tween 80 and the resulting mixture was thoroughly stirred.

After approximately 30 min, a 5M aqueous calcium chloride solution was added and the mixture was stirred for another 30 min. Subsequently, a 1M aqueous sodium phosphate solution was added and the mixture was further stirred. The mixture was continuously stirred over one day and night, and the resulting solution was freeze-dried over night to give desired calcium phosphate-coated retinoic acid nanoparticles (retinoic acid-CaPO₄ nanoparticles).

The resulting retinoic acid-CaPO₄ nanoparticles were also similar to the nanoparticles of Test Examples 1 and 2 in terms of particle size.

The retinoic acid nanoparticles coated with an inorganic salt of polyvalent metal thus obtained were analyzed in biological tests for their pharmacological activity and the effect of the particle size.

First, retinoic acid-CaCO₃ nanoparticles were tested for their ability to suppress the growth of melanoma cells. The nanoparticles were prepared by adding calcium chloride and sodium carbonate at a molar ratio of 1:1.0 to thereby deposit calcium carbonate coating on the surface of retinoic acid micelles.

### Test Example 5: in vitro experiment to determine the effect of CaCO₃ nanoparticles on B16 melanoma cells

It is a well-known fact that retinoic acid has an ability to suppress the growth of B16 melanoma cells. The following tests were conducted to determine if the growth of B16 melanoma cells could be suppressed by the retinoic acid-CaCO₃ nanoparticles obtained in the above-described Test Examples and how significant the suppressive effect would be as compared to non-nanoparticle retinoic acid alone. (Method)

B16 melanoma cells (2 x 10⁴ cells) were cultured in separate wells for 24 hours. To these wells, retinoic acid or the retinoic acid-CaCO₃ nanoparticles were added at different concentrations and the cells were cultured for additional 48 hours. Subsequently, the uptake of ³H-thymidine by the cells was measured for each well and the DNA synthesis was compared between the cells.

### (Results)

The results are shown in Fig. 3. The results indicate that the retinoic acid-CaCO₃ nanoparticles of the present invention show higher growth inhibition than non-nanoparticle retinoic acid alone with the difference becoming more significant at higher concentrations.

Thus, the retinoic acid-CaCO₃ nanoparticles of the present invention have been proved to be highly effective in the suppression of the growth of B16 melanoma cells.

### Test Example 6: in vivo experiment to determine kinetics of subcutaneously administered nanoparticles in blood in rats

### (1) Subcutaneous administration

### (Method)

³H-labelled retinoic acid and retinoic acid-CaCO₃ nanoparticles obtained from ³H-labelled retinoic acid micelles were subcutaneously administered to Wistar rats (7 week old/male). Blood samples were collected at intervals and analyzed for the retinoic acid level using a scintillation counter.

150nm retinoic acid-CaCO₃ nanoparticles (in average particle size) were used in the test.

As a control, retinoic acid micelles were used without being formed into nanoparticles.

### (Results)

Fig. 4 shows the comparison of the effect of subcutaneous administration between the retinoic acid-CaCO₃ nanoparticles (average particle size = approx. 150nm) and the non-nanoparticle retinoic acid micelles to serve as control.

As can be seen from the results, the non-nanoparticle retinoic acid micelles to serve as control released significant amounts of retinoic acid within about 1 hour after administration, whereas the blood level of retinoic acid was initially kept low and the release of retinoic acid was sustained over about 7 days period for the retinoic acid-CaCO₃ nanoparticles.

These results support the ability of the retinoic acid-CaCO₃ nanoparticles to release retinoic acid in a sustained manner and thus prove the effectiveness of the nanoparticles as a sustained-release preparation.

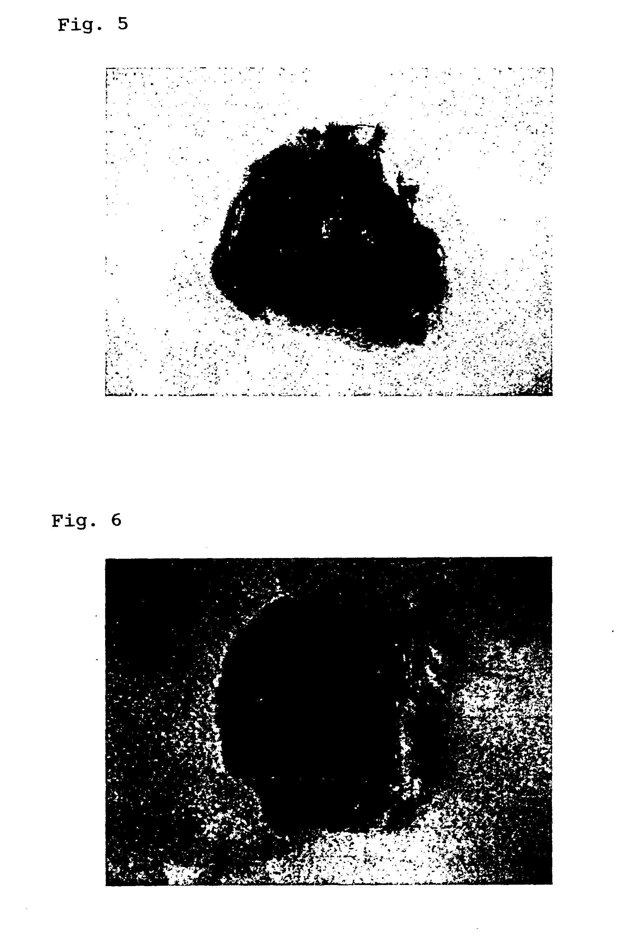

Figs. 5 and 6 show photographs of the site of application 10 days after administration of the non-nanoparticle retinoic acid micelles as control (Fig. 5) and the retinoic acid-CaCO₃ nanoparticles (Fig. 6). It is seen that the irritancy of retinoic acid caused inflammation at the site of application after the administration of the retinoic acid micelles, whereas no inflammation was induced after the administration of the retinoic acid-CaCO₃ nanoparticles, indicating reduced irritancy of retinoic acid.

These results indicate that the retinoic acid-CaCO₃ nanoparticles have reduced skin irritancy and are therefore suitable for use as external applications or cosmetics for skin application.

### (2) Topical application

The dorsal skin of mice (ddy strain/5week old/male) was clipped with electric clippers, and the ³H-labelled retinoic acid, as well as the retinoic acid-CaCO₃ nanoparticles and the retinoic acid-ZnCO₃ nanoparticles obtained from the ³H-labelled retinoic acid micelles, was mixed with a Vaseline base and was applied to the clipped area. Blood samples were collected at intervals and analyzed for the retinoic acid level using a scintillation counter.

The samples tested were as follows:
(a) Retinoic acid-CaCO₃ nanoparticles (average particle size = approx. 20 nm); and
   (b) Retinoic acid-ZnCO₃ nanoparticles (average particle size = approx. 20 nm).

Retinoic acid that was not formed into nanoparticles was mixed with a Vaseline base and used as control.

### (Results)

The results are shown in Table 7. As shown, the blood level of retinoic acid was significantly higher after topical application of the retinoic acid-CaCO₃ nanoparticles (average particle diameter = approx. 20 nm) or the retinoic acid-ZnCO₃ nanoparticles (average particle diameter = approx. 20 nm) mixed with Vaseline base than after topical application of the non-nanoparticle retinoic acid.

Thus, it has been shown that the pharmacological effect of the retinoic acid nanoparticles coated with an inorganic acid of polyvalent metal provided in accordance with the present invention can be enhanced by adjusting the average particle size of the nanoparticles to a very small size.

### Test Example 7: in vivo experiment to determine the effect of topical application of nanoparticles on the growth of epithelial cells in mice

### (Method)

The dorsal skin of mice (ddy strain/5week old/male) was clipped with electric clippers and a Vaseline based retinoic acid preparation (containing 0.1% retinoic acid) was applied to the clipped area 10 mg/cm² per day for 4 consecutive days. The epithelial thickness at the site of application was measured on Day 5.

The retinoic acid samples tested were as follows:
(a) Retinoic acid-CaCO₃ nanoparticles (average particle size = approx. 20 nm);
(b) Retinoic acid-ZnCO₃ nanoparticles (average particle size = approx. 20 nm);
(c) Retinoic acid-Ca nanoparticles obtained by adding calcium chloride to retinoic acid micelles to deposit calcium coating on the micelle surface (average particle size = approx. 20 nm);
(d) Retinoic acid-Zn nanoparticles obtained by adding zinc chloride to retinoic acid micelles to deposit zinc coating on the micelle surface (average particle size = approx. 20 nm); and
(e) Retinoic acid alone.

As controls, one group was given no treatment and one group was given Vaseline alone.

### (Results)

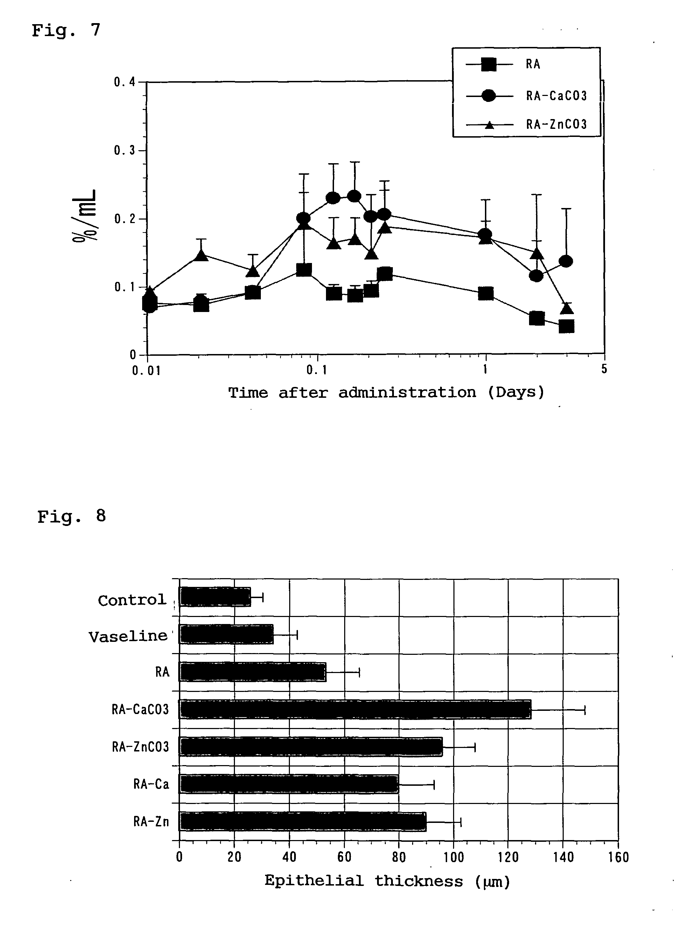

The results are shown in Fig. 8. As shown, the growth of epithelial cells was significantly faster and the increase in the epithelial thickness was significantly greater for the retinoic acid-CaCO₃ nanoparticles and the retinoic acid-ZnCO₃ nanoparticles than for the retinoic acid preparation.

The increase in the epithelial thickness was also greater for the retinoic acid-Ca nanoparticles (Ra-Ca), obtained by adding calcium chloride to retinoic acid micelles to deposit calcium coating on the micelle surface, and for the retinoic acid-Zn nanoparticles (RA-Zn), obtained by adding zinc chloride to retinoic acid micelles to deposit zinc chloride coating on the micelle surface, than for retinoic acid alone. This suggests that the retinoic acid micelles stabilized by metal halide or metal acetate coating can significantly facilitate the growth of epithelial cells as compared to the retinoic acid preparation. Such coated micelles are also encompassed by the scope of the present invention.

### Test Example 8: in vivo experiment to determine expression levels of HB-EGF m-RNA in mice

### (Method)

A Vaseline-based retinoic acid preparation (containing 0.1% retinoic acid) was applied to the pinna of the ear of mice (ddy strain, 5 week old/male) 30 mg/pinna/day for 4 consecutive days. The ear was excised on Day 5 and RNA was extracted using real-time PCR to determine the expression level of HB-EGF m-RNA.

Glyceraldehyde-3-phosphate dehydrogenase (GAPDH) m-RNA was also synthesized as a housekeeping gene and quantified to serve as a standard.

The retinoic acid samples tested were as follows:
(a) Retinoic acid-CaCO₃ nanoparticles (average particle size = approx. 20 nm);
(b) Retinoic acid-ZnCO₃ nanoparticles (average particle size = approx. 20 nm); and
(c) Retinoic acid alone.

### (Results)

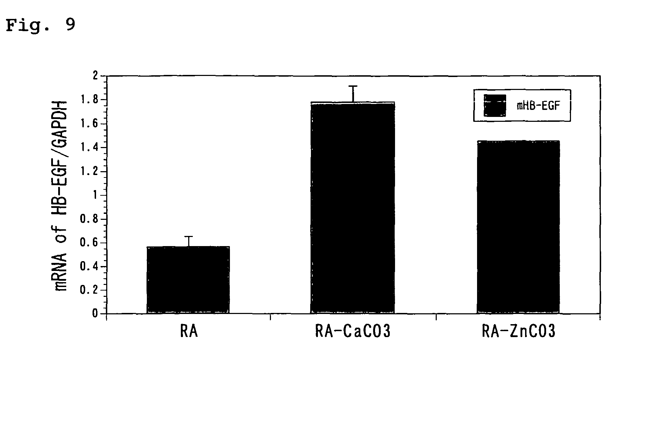

The results are shown in Fig. 9. As shown, the expression level of HB-EGF m-RNA was significantly higher for the size-adjusted retinoic acid-CaCO₃ nanoparticles (average particle size = approx. 20 nm) and the retinoic acid-ZnCO₃ nanoparticles (average particle size = approx. 20 nm) of the present invention than for retinoic acid alone.

The fact that expression of mRNA of HB-EGF, an epithelial growth factor, was enhanced by the retinoic acid nanoparticles coated with an inorganic acid of polyvalent metal of the present invention suggests that the nanoparticles have high ability to facilitate the skin regeneration.

### INDUSTRIAL APPLICABILITY

As set forth, the present invention provides retinoic acid nanoparticles coated with an inorganic salt of polyvalent metal and sized 5 to 300 nm. The nanoparticles are obtained by depositing a coating of a polyvalent metal inorganic salt on the surface of retinoic acid micelles. The nanoparticles of the present invention have high skin permeability and can be dissolved in water to form a stable clear solution that can be formulated into preparations for subcutaneous and intravenous administration. The inorganic salt coating helps reduce the irritancy of retinoic acid, so that the nanoparticles do not cause inflammation or tumor formation at the site of application.

Effectively making use of the pharmacological effect of retinoic acid, the retinoic acid nanoparticles coated with an inorganic acid of polyvalent metal of the present invention are expected to find wide applications in external preparations, cosmetics and regenerative medicine and are thus of significant medical importance.

## Claims

1. A method for adjusting a particle size of retinoic acid nanoparticles comprising micelles of retinoic acid coated with an inorganic salt of polyvalent metal, the method comprising:
dispersing retinoic acid dissolved in a lower alcohol in an aqueous alkali solution;
adding a nonionic surfactant to the dispersion to form a mixed micelle;
adding to the micelle a halide or acetate of divalent metal along with a carbonate or phosphate of alkali metal so that a molar ratio of the former to the latter is 1:0 to 1:1.0, thereby depositing a coating of the inorganic salt of the polyvalent metal on a surface of the micelle; and
adjusting an average particle size of the resulting nanoparticles to 5 to 300 nm.

2. The method for adjusting a particle size of retinoic acid nanoparticles coated with an inorganic salt of polyvalent metal according to claim 1, wherein the coating of the inorganic salt of the polyvalent metal is calcium carbonate, zinc carbonate, or calcium phosphate coating.

3. The method for adjusting a particle size of retinoic acid nanoparticles coated with an inorganic salt of polyvalent metal according to claim 1, wherein the halide or acetate of divalent metal is calcium halide, zinc halide, calcium acetate or zinc acetate.

4. The method for adjusting a particle size of retinoic acid nanoparticles coated with an inorganic salt of polyvalent metal according to claim 3, wherein the calcium halide or the zinc halide is selected from the group consisting of calcium chloride, calcium bromide, calcium fluoride, calcium iodide, zinc chloride, zinc bromide, zinc fluoride and zinc iodide.

5. The method for adjusting a particle size of retinoic acid nanoparticles coated with an inorganic salt of polyvalent metal according to claim 1, wherein the carbonate or phosphate of alkali metal is selected from the group consisting of sodium carbonate, potassium carbonate, sodium phosphate, and potassium phosphate.

6. The method for adjusting a particle size of retinoic acid nanoparticles coated with an inorganic salt of polyvalent metal according to claim 1, wherein the lower alcohol is methanol or ethanol.

7. The method for adjusting a particle size of retinoic acid nanoparticles coated with an inorganic salt of polyvalent metal according to claim 1, wherein the nonionic surfactant is polyoxyethylene (20) sorbitan monooleate, polyoxyethylene (20) sorbitan monolaurate, polyoxyethylene (20) sorbitan monostearate, polyoxyethylene (20) sorbitan monopalmitate, polyoxyethylene (20) sorbitan trioleate, polyoxyethylene (8) octylphenylether, polyoxyethylene (20) cholesterol ester, polyoxyethylene (30) cholesterol ester or polyoxyethylene hydrogenated castor oil.

8. The method for adjusting a particle size of retinoic acid nanoparticles coated with calcium carbonate claimed in claim 1, comprising micelles of retinoic acid coated with calcium carbonate, the method comprising:
dispersing retinoic acid dissolved in a lower alcohol in an aqueous alkali solution;
adding a nonionic surfactant to the dispersion to form a mixed micelle;
adding to the micelle calcium chloride and sodium carbonate so that a molar ratio of the former to the latter is 1:0 to 1:1.0, thereby depositing a coating of calcium carbonate on a surface of the micelle; and
adjusting the average particle size of the resulting nanoparticles to 5 to 300 nm.

9. The method for adjusting a particle size of retinoic acid nanoparticles coated with calcium carbonate according to claim 8, wherein the lower alcohol is methanol or ethanol.

10. The method for adjusting a particle size of retinoic acid nanoparticles coated with calcium carbonate according to claim 8, wherein the nonionic surfactant is polyoxyethylene (20) sorbitan monooleate, polyoxyethylene (20) sorbitan monolaurate, polyoxyethylene (20) sorbitan monostearate, polyoxyethylene (20) sorbitan monopalmitate, polyoxyethylene (20) sorbitan trioleate, polyoxyethylene (8) octylphenylether, polyoxyethylene (20) cholesterol ester, polyoxyethylene (30) cholesterol ester or polyoxyethylene hydrogenated castor oil.

11. Retinoic acid nanoparticles coated with an inorganic salt of polyvalent metal and having an average particle size of 5 to 300 nm, obtained by the adjusting method according to any of claims 1 to 7.

12. Calcium carbonate-coated retinoic acid nanoparticles obtained by the adjusting method according to any of claims 8 to 10 and having an average particle size of 5 to 300 nm.

13. Calcium carbonate-coated nanoparticles having an average particles size of 5 to 300 nm and comprising retinoic acid micelles coated with calcium carbonate.

14. Zinc carbonate-coated nanoparticles having an average particles size of 5 to 300 nm and comprising retinoic acid micelles coated with zinc carbonate.

15. Calcium phosphate-coated nanoparticles having an average particles size of 5 to 300 nm and comprising retinoic acid micelles coated with calcium phosphate.
